Europäisches Patentamt

European Patent Office

Office européen des brevets

(19)

(11) Veröffentlichungsnummer: 0 183 029
A2

(12) # EUROPÄISCHE PATENTANMELDUNG

(21) Anmeldenummer: 85113047.6

(22) Anmeldetag: 15.10.85

(51) Int. Cl.⁴: **C 07 C 31/20**
C 07 C 29/00, C 07 C 29/10

(30) Priorität: 24.11.84 DE 3442937

(43) Veröffentlichungstag der Anmeldung:
04.06.86 Patentblatt 86/23

(84) Benannte Vertragsstaaten:
AT CH DE FR GB IT LI NL SE

(71) Anmelder: Degussa Aktiengesellschaft
Weissfrauenstrasse 9
D-6000 Frankfurt am Main 1(DE)

(72) Erfinder: Siegmeier, Rainer, Dr. Dipl.-Chem.
Egenolffstrasse 4
D-6000 Frankfurt(DE)

(72) Erfinder: Prescher, Günter, Dr. Dipl.-Chem.
Liesingstrasse 2
D-6450 Hanau 9(DE)

(72) Erfinder: Maurer, Helmut
In den Steinäckern 5
D-6458 Rodenbach(DE)

(72) Erfinder: Hering, Günter
Birkenberg 48
D-8755 Alzenau 4(DE)

(54) Kontinuierliches Verfahren zur Herstellung von 1,2-Pentandiol.

(57) Kontinuierliche Herstellung von 1,2 Pentandiol, ausgehend von Penten, das in völlig homogener Phase mit benzolischer Perpropionsäure epoxidiert wird zu 1-Pentenoxid, das seinerseits ohne Isolierung direkt verseift wird. Hohe Ausbeuten und hoher Reinheitsgrad von 1,2-Pentandiol.

EP 0 183 029 A2

./...

Fig.1

D e g u s s a    Aktiengesellschaft
Weissfrauenstraße 9, 6000 Frankfurt 1

Kontinuierliches Verfahren zur Herstellung von
1,2-Pentandiol

Beschreibung

Die Erfindung betrifft die kontinuierliche Herstellung von 1,2-Pentandiol aus Penten durch dessen Oxidation zu 1,2-Pentenoxid und Verseifung dieses Epoxids mit Wasser zu 1,2-Pentandiol.

1,2-Pentandiol dient als Zwischenprodukt zur Herstellung von Fungiziden.

Hinweise zur Herstellung von 1,2-Pentandiol finden sich z.B. in der DE-PS 2 205 023, die ein einstufiges Verfahren zur gleichzeitigen Epoxidierung und Verseifung von höheren Olefinen, d.h. von Olefinen, die bevorzugt mindestens 4 Kohlenstoffatome haben sollen, beschreibt. Dieses einstufige Verfahren verwendet wäßrige Percarbonsäurelösungen sowohl zur Epoxidation als auch zur Verseifung, benötigt aber erhebliche Wassermengen, die besonders aufwendig bei der kontinuierlichen Durchführung sind.

Nach der US-PS 3 475 499 wird ein zweistufiges Verfahren zur Herstellung von 1,2-Epoxiden und Verseifung zu den entsprechenden Diolen beschrieben, bei dem die Epoxidation mit Hydroperoxiden in Gegenwart von organischen Lösungsmitteln wie Kohlenwasserstoffen kontinuierlich durchgeführt werden kann. Diese Kohlenwasserstoffe sollen jedoch vor der Verseifung bevorzugt abdestilliert werden.

Nach dieser Patentschrift werden die bei der Destillation des Epoxidierungsgemisches anfallenden epoxidhaltigen Sümpfe mit Wasser in Gegenwart von alkalischen oder sauren Katalysatoren verseift. Die Ausbeute an Epoxid liegt nach Beispiel 1, das einen $C_{11}$ -$C_{15}$-Olefinschnitt betrifft, nur um 45 %; sie wird zusammen mit dem nicht umgesetzten Olefin verseift.

Obwohl die Selektivität hierbei hoch ist, ist die Menge an reinem Diol, bedingt durch die geringe Epoxidausbeute, mäßig und ist technisch uninteressant.

Aufgabe der Erfindung ist es also, ein technisch nicht aufwendiges kontinuierliches Verfahren durch Epoxidieren von 1,2-Penten und anschließende Verseifung des entstandenen Epoxids im wäßrigen Milieu mit einer technisch befriedigenden Ausbeute und sehr guter Selektivität anzugeben.

Es wurde nun gefunden, daß sich diese Aufgabe lösen läßt, wenn man in einer ersten Stufe Penten mit einer benzolischen Lösung von Perpropionsäure, in der gegebenenfalls noch Propionsäure vorhanden ist, bei einem Molverhältnis von Penten zu Perpropionsäure wie 0,5 bis 5 : 1 bei Temperaturen von 20 bis 110 $^{\circ}$C und einem Druck von 1 bis 10 bar umsetzt, das Epoxidationsgemisch fraktioniert destilliert und die am leichtesten flüchtige Fraktion, die hauptsächlich aus Penten besteht, in die Epoxidation mit der Perpropionsäurelösung zurückführt, die mittelflüchtige Fraktion, die in der Hauptsache aus Pentenoxid und Benzol besteht, in die Mitte einer Verseifungskolonne einführt, dort mit einer zur Verseifung des Pentenoxids ausreichenden Menge Wasser in Gegenwart eines sauren Katalysators bei 30 bis 150 $^{o}$C, vorzugsweise bei 60 bis 80 $^{o}$C, sowie bei 1 bis 5 bar verseift, über Kopf ein Benzol-Wassergemisch abnimmt, dieses Gemisch in Wasser, das in die Verseifungskolonne zurückgeführt, und Benzol auftrennt und am Boden der Kolonne 1,2-Pentandiol in wäßrig saurer Lösung abzieht und daß man die schwerstflüchtige Fraktion am Boden der Fraktionierkolonne für das Epoxidationsgemisch entnimmt.

Die Perpropionsäurelösungen in Benzol enthalten im allgemeinen 10 bis 30 Gewichtsprozent Perpropionsäure, bevorzugt 20 Gewichtsprozent.

Für das einzusetzende Penten war das handelsübliche Penten mit einem Gehalt an 1-Penten von etwa 96 % recht günstig; falls erwünscht, kann auch Penten mit einem noch höheren oder niederen Gehalt an 1-Penten verwendet werden. Penten wird bevorzugt in flüssiger Phase unter einem geringen Überdruck eingesetzt.

Bevorzugte Molverhältnisse von Penten zu Perpropionsäure liegen bei 0,7 bis 5 : 1, vor allem bei 1 bis 3 : 1.

Als günstig haben sich besonders Temperaturen von 20 bis 100 $^{o}$C, vor allem von 55 bis 60 $^{o}$C erwiesen, ebenso Drucke von 1 bis 2 bar.

Die Epoxidation wird in bekannten Reaktoren durchgeführt, bevorzugt in zwei hintereinandergeschalteten Reaktoren. Sollte es nötig sein, kann noch ein Nachreaktor eingeschaltet werden. Als Reaktoren kommen die üblichen Rührkessel, Röhrenreaktoren oder Schlaufenreaktoren in Frage. Bekanntlich ist eine rasche Trennung von Epoxid und gebildeter Carbonsäure sehr erwünscht. Das Epoxidationsgemisch wird daher in drei Fraktionen destillativ aufgeteilt: Die flüchtigste Fraktion, die hauptsächlich aus Penten besteht, wird in die Epoxidation zurückgeführt, die Fraktion mit mittlerer Flüchtigkeit ist in der Hauptsache Pentenoxid in Benzol und wird in der Verseifungsstufe weiterbehandelt, während die schwerstflüchtige Fraktion aus Propionsäure und hochsiedenden Nebenprodukten besteht. Eine erneute Trennung in Propionsäure und die Nebenprodukte, z.B. durch destillative Aufarbeitung, und Rückführung der Propionsäure in die Perpropionsäureherstellung ist möglich.

Die fraktionierte Destillation des Epoxidationsgemisches kann in einer einzigen Kolonne durchgeführt werden. Besonders günstig ist es aber zweistufig, also in zwei Kolonnen zu arbeiten, die hintereinandergeschaltet sind, und die leichter flüchtige Fraktion der ersten Kolonne, die hauptsächlich aus Penten, Pentenoxid und Benzol besteht, in einer zweiten Kolonne weiter zu fraktionieren. Die Sumpffraktion der zweiten Kolonne wird dann in die Verseifungsstufe geleitet.

Als Kolonnen für die Destillation des Epoxidgemisches wie für die Verseifung kommen übliche Destillationskolonnen in Frage, die man bevorzugt bei Atmosphärendruck, Über- oder Unterdruck betreibt.

Diese Sumpffraktion aus hauptsächlich Pentenoxid und Benzol wird in die Mitte der Verseifungskolonne eingeführt und das Wasser, das den sauren Katalysator enthält, nahe dem Kopf der Kolonne eingegeben. Das bei der Destillation am Kopf abgenommene Gemisch aus Benzol und Wasser ist ein Azeotrop mit etwa 91 Gewichtsprozent Benzol, das bei 69 $^{o}$C siedet.

Das in die Kolonne eingeführte Wasser dient also nicht nur zur Verseifung des Pentenoxids, sondern gleichzeitig auch zur Entfernung des Benzols. Es muß also mindestens so viel Wasser anwesend sein, daß diese beiden Forderungen erfüllt werden können.

Es erwies sich, daß ein Benzol-Wasser-Verhältnis von 5 : 1 bis 1 : 1 Gewichtsteilen recht günstig war. Sehr geeignet war ein Verhältnis von 1,5 bis 2 : 1 Gewichtsteil.

Will man die Wassermenge auch in Bezug auf die zu verseifenden Menge Pentenoxid setzen, so haben sich auch hier Mengen von 1 bis 5 Gewichtsteilen Wasser auf 1 Gewichtsteil 1,2-Pentenoxid bewährt.

Das am Kopf der Kolonne übergegangene Azeotrop wird in Benzol und Wasser getrennt, von denen das Wasser direkt in die Verseifungskolonne, das Benzol - wenn erwünschtin die Herstellung der benzolischen Perpropionsäurelösung oder eventuell in die Epoxidationsstufe, zurückgeführt wird.

Der saure Katalysator liegt in Mengen von 0,05 bis 5 Gewichtsprozent im Wasser vor.

Die als saure Katalysatoren verwendeten Stoffe sind an sich hierfür bekannt. Sie können sowohl anorganische als auch organische Verbindungen sein. So kommen Mineralsäuren, wie organische Säuren in Frage. Bevorzugt sind von

6      84 0118029

den Mineralsäuren Schwefelsäure, Salzsäure oder Orthophosphorsäure; von den organischen Säuren Ameisen-, Essig-
oder Propionsäure oder auch iso-Buttersäure bzw. Methansulfonsäure. Besonders bevorzugt ist Schwefelsäure.

Das als Sumpf der Verseifungskolonne erhaltene 1,2-Pentandiol in wäßrig saurer Lösung wird bevorzugt einer Nachbehandlung durch Neutralisation und anschließende Destillation unterworfen. Das Neutralisieren kann z.B. mit Hilfe
eines basischen Ionenaustauschers erfolgen; das dabei erhaltene neutralisierte Pentandiol wird zur Entfernung von
Wasser und hochsiedenden Nebenprodukten fraktioniert destilliert und als Seitenstrom in hochreiner Form der Destillationskolonne entnommen. Das dabei als Kopfprodukt
anfallende Wasser kann der Verseifungsstufe zugeführt
werden.

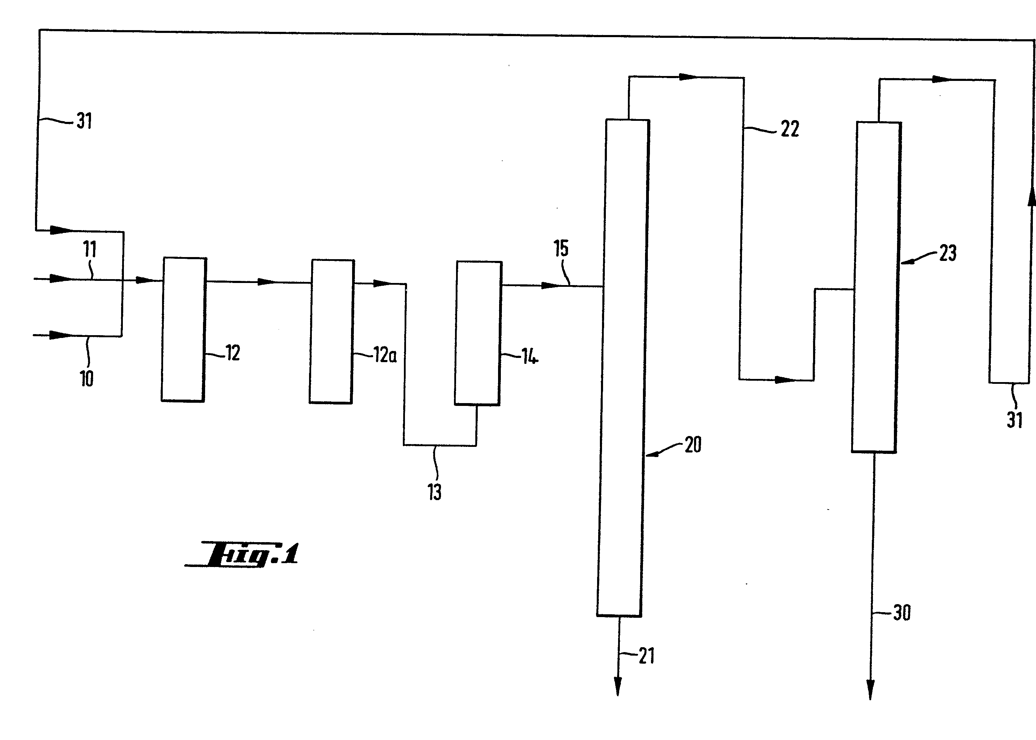

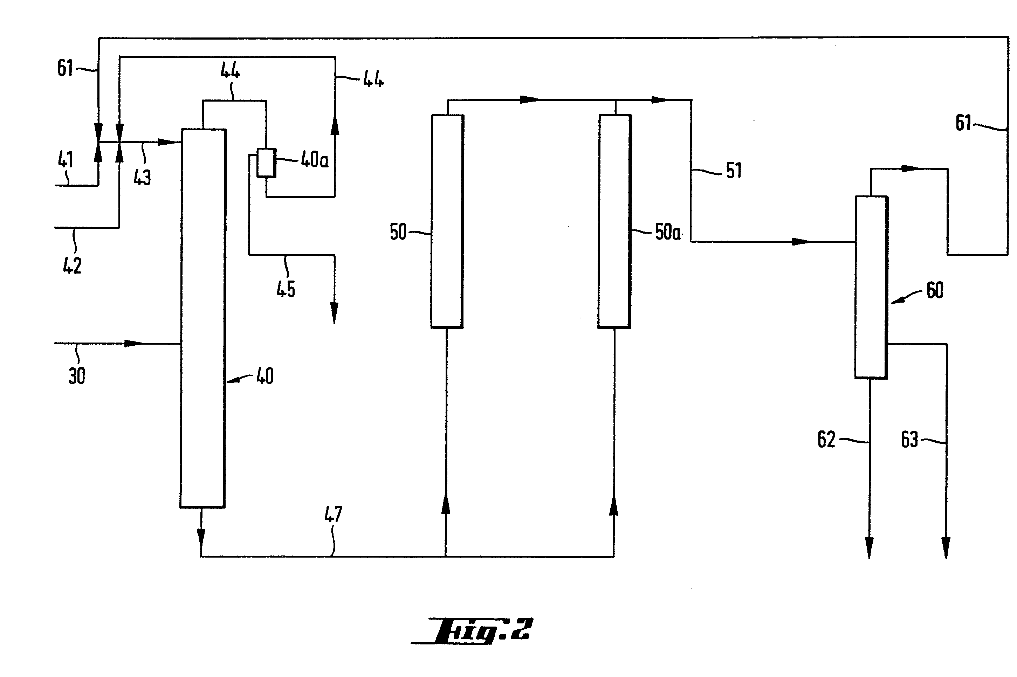

Die Erfindung wird anhand der Abbildungen 1 und 2 näher
erläutert; hiervon betrifft Abbildung 1 die Epoxidation
von Penten mit benzolischer Perpropionsäurelösung, sowie
Aufarbeitung des Epoxidationsgemisches; Abbildung 2 die
Verseifung der Epoxidlösung und Reindarstellung des Produkts.

Die Anlage besteht aus zwei Reaktoren 12 und 12a, die mit
einer Lösung von Perpropionsäure in Benzol über die Zuleitung 10 und mit Frisch-Penten über Zuleitung 11 sowie Rück-
Penten über die Rückführungsleitung 31 beschickt werden.
Das Reaktionsgemisch wird über eine Zuleitung 13 - falls
nötig - einem Nachreaktor 14 zugeführt. Aus Reaktor 12a
bzw. Nachreaktor 14 wird das Reaktionsgemisch einer Destillationskolonne 20 zugeführt. Von dieser Kolonne wird
eine schwererflüchtige Fraktion über Leitung 21 abgezogen,
die aus Propionsäure und hochsiedenden Nebenprodukten besteht.

Die leichtersiedende Fraktion der Destillationskolonne 20 wird über die Leitung 22 in eine zweite Destillationskolonne 23 eingespeist. Die leichtersiedende Fraktion der Kolonne 23, die ausschließlich Penten enthält, wird über die Rückführungsleitung 31 den Reaktoren 12/12a zugeführt. Die schwerflüchtige Fraktion der Destillationskolonne 23 enthält fast ausschließlich Pentenoxid und Benzol. Sie wird über die Zuleitung 30 der Stufe 2 zugeführt.

Die in der Stufe 1 hergestellte Lösung von Pentenoxid in Benzol wird über die Zuleitung 30 in die Mitte einer Destillationskolonne 40 eingespeist. In den oberen Teil der Kolonne 40 werden über die Zuleitung 41 Frischwasser, die Rückführungsleitung 61 und 44 Rückwasser und über die Leitung 42 Schwefelsäure gemeinsam dosiert. Die leichtersiedende Fraktion der Destillationskolonne 40 besteht aus Wasser, das über die Rückführungsleitung 4♯ wieder der Kolonne zugeführt wird, und Benzol, das in einem Scheidegefäß 40a vom Wasser getrennt und über die Leitung 45 abgeführt werden kann. Die schwerflüchtige Fraktion der Kolonne 40, die hauptsächlich aus verdünnter Schwefelsäure und Pentandiol besteht, wird über die Leitung 47 den Ionenaustauschern 50 zugeführt, die wechselseitig betrieben werden können. Von den Austauschern 50 wird die Lösung über die Leitung 51 einer Destillationsstufe 60 zugeführt. Die leichtersiedende Fraktion, die fast ausschließlich Wasser enthält, wird über die Rückführungsleitung 61 der Destillationskolonne 40 zugeführt. Im unteren Teil der Destillationsstufe 60 wird in einem Seitenstrom 63 das Endprodukt Pentandiol-1,2 abgezogen. Die hochsiedenden Abfallprodukte werden über die Leitung 62 abgeführt.

Der technische Fortschritt des vollkontinuierlichen erfindungsgemäßen Verfahrens liegt einmal in der Möglichkeit, die Epoxidation in vollständig homogener Phase bei Normaldruck oder nur leichtem Überdruck, bei dem der Siedepunkt der Lösung nicht erreicht wird, bei hohem Umsatz und hoher Selektivität durchzuführen. Das hierbei gebildete Epoxid wird ohne Isolierung direkt der Verseifung unterworfen und Pentandiol in sehr guten Ausbeuten, bezogen auf Penten, erhalten; außerdem ist sein Reinheitsgrad hoch. Im allgemeinen werden Perpropionsäureumsätze um 99 % erreicht und Expodidationsausbeuten von etwa 93 %, bezogen auf umgesetztes Penten. Die Ausbeuten in der Verseifungsstufe liegen für den quantitativen Epoxidumsatz i. a. bei 99 %. Das Endprodukt Pentandiol wird i. a. in einem Reinheitsgrad von größer als 99 % erhalten.

Die Verseifung wird mit wesentlich geringeren Mengen Wasser als nach dem Verfahren der DE-PS 2 205 023 durchgeführt. Es kommt demgegenüber hinzu, daß nach dem erfindungsgemäßen Verfahren in der Verseifungsreaktion die freiwerdende Reaktionswärme für die gleichzeitige Destillation des Benzols ausgenutzt wird. Hierdurch wird ein Teil der notwendigen zuzuführenden Energie eingespart; es kann daher auf eine zusätzliche Trennungsstufe verzichtet werden.
Außerdem liegt ein völlig geschlossenes System vor, bei dem die Einsatz- bzw. Hilfsstoffe wie Penten, Propionsäure, Benzol und Wasser im Kreislauf gefahren werden und das System nicht verlassen. Die Erfindung wird in dem nachfolgenden Beispiel (kontinuierliche Durchführung) und Vergleichsbeispiel (diskontinuierliche Durchführung) näher erläutert.

Beispiel (kontinuierlich)

In einem Versuch von 70 Stunden Dauer werden pro Stunde 100 g Frisch-Penten und etwa 52 g Rück-Penten sowie ca. 600 g einer 20 gewichtsprozentigen Lösung von Perpropionsäure in Benzol in die Reaktoren 12 und 12 a eingespeist, die auf einer Temperatur von 55 bis 60 $^{\circ}$C und 1,0 bar gehalten werden. Den Nachreaktor 14 verlassen etwa 753 g Reaktionsgemisch pro Stunde, die in der Destillationskolonne 20 in 189 g Propionsäure, die noch hochsiedende Nebenprodukte enthält, und 564 g der leichtersiedenden Fraktion aufgetrennt werden.

Die leichtsiedende Fraktion wird in der Kolonne 23 erneut destilliert. Über Kopf werden etwa 52 g Rück-Penten abgenommen, das über Leitung 31 in die Reaktoren 12 und 12 a zurückgeleitet wird.Das Sumpfprodukt, das fast ausschließlich aus Pentenoxid und Benzol besteht (512 g), wird in der Stufe 2 über Leitung 30 der Kolonne 40 zugeführt. In diese werden im oberen Teil über Leitung 43 insgesamt 214 g Wasser, das 0,1 Gewichtsprozent Schwefelsäure enthält, eindosiert. Über Kopf werden stündlich – über Leitung 44 – 381 g Benzol sowie 34 g Wasser abgenommen, im Scheidegefäß 40 a in Benzol, das über Leitung 45 abgeführt, und in Wasser, das über Leitung 46 in Leitung 43 und Kolonne 40 rückgeführt wird, aufgetrennt.

Im Sumpf der Kolonne 40 fallen etwa 288 g einer wäßrigen Lösung von Pentandiol, die noch Schwefelsäure (ca. 0,07 Gewichtsprozent) enthält, an. Diese saure Lösung wird in den Ionenaustauschern 50 und 50 a neutralisiert und anschließend in Kolonne 60 destilliert. Man erhält etwa 130 g Pentandiol pro Stunde und 154 g Wasser, das wieder der Kolonne 40 über Leitung 61 zugeführt wird. Nach der ersten Stufe lag Pentenoxid in einer Ausbeute von 93,6 %, bezogen auf umgesetztes Penten, vor. Die Verseifungsausbeute in Stufe 2 betrug 99 %, bezogen auf eingesetztes Epoxid. Die Reinheit des Endproduktes beträgt 99,2 %.

Als Ionenaustauscher kommen infrage: basische Ionenaustauscher, z. B. vernetzte Polystyrole mit Trialkylammoniumendgruppen oder Phenol-Formaldehydkondensate mit Alkylaminen.

Vergleichsbeispiel (diskontinuierlich)

In einem Dreihalskolben (1000 ml), der mit Innenthermometer und Rückflußkühler ausgerüstet ist, werden 112 g Pentenoxid, 224 g Wasser und 397 g Benzol vorgelegt, mit 0,1 Gewichtsprozent Schwefelsäure, bezogen auf das eingesetzte Wasser, versetzt und zum Sieden erhitzt.

Nach einer Reaktionszeit von 8 Stunden wurden der Epoxidumsatz, die Ausbeute und die Menge der gebildeten hochsiedenden Nebenprodukte titrimetrisch und gaschromatographisch ermittelt.

Bezogen auf eingesetztes Pentenoxid lag die Ausbeute bei 88 % und die Reinheit betrug 92,8 %.

D e g u s s a    Aktiengesellschaft
Weissfrauenstraße 9, 6000 Frankfurt 1

Kontinuierliches Verfahren zur Herstellung von
1,2-Pentandiol

Patentansprüche

1. Kontinuierliches Verfahren zur Herstellung von 1,2-Pentandiol, ausgehend von Penten, dessen Oxidation zu 1,2-
Pentenoxid unter Verwendung von Perpropionsäure und Verseifung dieses Epoxids mit Wasser bei mäßiger Temperatur
und mäßigem Druck, dadurch gekennzeichnet, daß man in
einer ersten Stufe Penten mit einer benzolischen Lösung
von Perpropionsäure, in der gegebenenfalls noch Propionsäure vorhanden ist, bei einem Molverhältnis von Penten
zu Perpropionsäure wie 0,5 bis 5 : 1 bei Temperaturen
von 20 bis 110 $^{\circ}$C und einem Druck von 1 bis 10 bar umsetzt, das Epoxidationsgemisch fraktioniert destilliert
und die am leichtesten flüchtige Fraktion, die hauptsächlich aus Penten besteht, in die Epoxidation mit der
Perpropionsäurelösung zurückführt, die mittelflüchtige
Fraktion, die in der Hauptsache aus Pentenoxid und Benzol besteht, in die Mitte einer Verseifungskolonne einführt, dort mit einer zur Verseifung des Pentenoxids ausreichenden Menge Wasser in Gegenwart eines sauren Katalysators bei 30 bis 150 $^{\circ}$C, vorzugsweise bei 60 bis 80 $^{\circ}$C,

sowie bei 1 bis 5 bar verseift, über Kopf ein Benzol-Wassergemisch abnimmt, dieses Gemisch in Wasser, das in die Verseifungskolonne zurückgeführt, und Benzol auftrennt und am Boden der Verseifungskolonne 1,2-Pentandiol in wäßrig saurer Lösung abzieht und daß man die schwerstflüchtige Fraktion am Boden der Fraktionierkolonne für das Epoxidationsgemisch entnimmt.

2. Verfahren nach Anspruch 1, dadurch gekennzeichnet, daß man eine benzolische Lösung mit 10 bis 30 Gewichtsprozent Perpropionsäure in der ersten Stufe einsetzt.

3. Verfahren nach den Ansprüchen 1 und 2, dadurch gekennzeichnet, daß man Penten zu Perpropionsäure im Molverhältnis von 0,7 bis 6 : 1, bevorzugt 1 bis 3 : 1, in der ersten Stufe verwendet.

4. Verfahren nach den Ansprüchen 1 bis 3, dadurch gekennzeichnet, daß man in der ersten Stufe bei Temperaturen von 20 bis 100 $^{\circ}$C und Drucken von 1,0 bis 2 bar arbeitet.

5. Verfahren nach den Ansprüchen 1 bis 4, dadurch gekennzeichnet, daß man das Epoxidationsgemisch aus Penten und Perpropionsäure zweistufig auftrennt, und zwar in einer ersten Destillationskolonne in eine hochsiedende Fraktion, bestehend aus Proponsäure, die ggf. aufgearbeitet werden kann, und Nebenprodukte und eine leichter flüchtige Fraktion, bestehend aus Penten, Pentenoxid und Benzol, und dann in einer zweiten Destillationskolonne diese leichter flüchtige Fraktion erneut in Penten, das in die Reaktionsstufe zurückgeführt, und in eine aus Pentenoxid und Benzol bestehende Fraktion, die in die Verseifungskolonne geleitet wird, fraktioniert.

6. Verfahren nach den Ansprüchen 1 bis 5, dadurch gekennzeichnet, daß man 1 bis 5 Gewichtsteile Wasser, das 0,05 bis 5 Gewichtsprozent eines sauren Katalysators enthält, pro Gewichtsteil 1,2-Pentenoxid, in der zweiten Stufe einsetzt.

7. Verfahren nach den Ansprüchen 1 bis 6, dadurch gekennzeichnet, daß man die Verseifung in einem Medium durchführt, dessen Benzol-Wasser-Verhältnis zwischen 5 : 1 bis 1 : 1 Gewichtsteilen liegt.

8. Verfahren nach den Ansprüchen 1 bis 7, dadurch gekennzeichnet, daß man als sauren Katalysator Schwefelsäure, Salzsäure oder Phosphorsäure einsetzt.

9. Verfahren nach den Ansprüchen 1 bis 7, dadurch gekennzeichnet, daß man als sauren Katalysator Ameisen-, Essig oder Propionsäure, iso-Buttersäure oder Toluolsulfonsäure verwendet.

Fig.1

0183029

Fig.2